Europäisches Patentamt

European Patent Office

Office européen des brevets

(11) Publication number: **0 211 899 B1**

# EUROPEAN PATENT SPECIFICATION

(45) Date of publication of patent specification: **23.09.92**

(51) Int. Cl.5: **C07K 3/12**, C07K 15/00, C12N 15/00, C12P 21/02, A61K 37/02, //C12Q1/68, (C12P21/02,C12R1:91)

(21) Application number: **86901265.8**

(22) Date of filing: **05.02.86**

(86) International application number: **PCT/US86/00251**

(87) International publication number: **WO 86/04587 (14.08.86 86/18)**

The file contains technical information submitted after the application was filed and not included in this specification

(54) **PURIFICATION OF NATIVE COLONY STIMULATING FACTOR-1.**

(30) Priority: **05.02.85 US 698358**

(43) Date of publication of application: **04.03.87 Bulletin 87/10**

(45) Publication of the grant of the patent: **23.09.92 Bulletin 92/39**

(84) Designated Contracting States: **AT BE CH DE FR GB IT LI LU NL SE**

(56) References cited:
**GB-A- 2 134 528**
**US-A- 4 438 032**
**US-A- 4 504 586**

**Chemical Abstracts, volume 97, no. 9, 30 August 1982, Columbus, Ohio, US A. Waheed et al.: "Purification of colony-stimulating factor by affinity chromatography", see page 486, abstract 70645N**

(73) Proprietor: **CETUS ONCOLOGY CORPORATION**
**1400 Fifty-Third Street**
**Emeryville California 94608(US)**

Proprietor: **ALBERT EINSTEIN COLLEGE OF MEDICINE OF YESHIVA UNIVERSITY**
**1300 Morris Park Avenue**
**Bronx New York 10461(US)**

(72) Inventor: **McGROGAN, Michael, P.**
**707 Curtis Street, No. 8**
**Albany, CA 94706(US)**
Inventor: **WILSON, Kenneth**
**2249 Lomond Lane**
**Walnut Creek, CA 94598(US)**
Inventor: **STRICKLER, James, E.**
**101 Pennsylvania Avenue**
**Havertown, PA 19083(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Chemical Abstracts, volume 100, no. 7, 13 February 1984, Columbus, Ohio, US Wang, F.F. et al.: "Purification of a human urinary colony stimulating factor", see page 62, abstract 45417r

Methods in Enzymology, volume 116, 1985 Academic Press, US E.R. Stanley: "The macrophage colony-stimulating factor, CSF-1" pages 564-587

Proc. Natl. Acad. Sci. USA, volume 82, July 1985, Washington, US C.M. Ben-Avram et al.:"Amino-terminal amino acid sequence of murine colony-stimulating factor 1", pages 4486-4489

Nature, volume 309, 28 June 1984 N.M. Gough et al.: "Molecular cloning of cDNA encoding a murine haematopoietic growth regulator, granulocyte-macrophage colony stimulating factor", pages 763-767

Science, volume 230, 18 October 1985, E.S. Kawasaki et al.: "Molecular cloning of a complementary DNA encoding human macrophage-specific colony-stimulating factor (CSF-1), pages 291-296

Inventor: **BOOSMAN, Albert**
**3073 Vessing Road**
**Pleasant Hill, CA 94523(US)**
Inventor: **WARREN, Mary, Kim**
**486 Joaquin Avenue**
**San Leandro, CA 94577(US)**
Inventor: **STANLEY, Richard, E.**
**1300 Morris Avenue**
**Bronx, NY 10461(US)**

(74) Representative: **Bizley, Richard Edward et al**
**HEPWORTH LAWRENCE BRYER & BIZLEY**
**2nd Floor Gate House South West Gate**
**Harlow, Essex CM20 1JN(GB)**

## Description

The present invention relates to the purification of proteins, to the products of such purification, and to DNA probes constructed therefrom. More specifically, the invention relates to the purification and sequencing of human colony stimulating factor-1 (CSF-1).

The ability of certain factors produced in very low concentration in a variety of tissues to stimulate the growth and development of bone marrow progenitor cells into granulocytes and/or macrophages has been known for nearly 15 years. The presence of such factors in sera, urine samples, and tissue extracts from a number of species is demonstrable using an in vitro assay which measures the stimulation of colony formation by bone marrow cells plated in semi-solid culture medium. There is no known in vivo assay. Because these factors induce the formation of such colonies, the factors collectively have been called Colony Stimulating Factors (CSF).

More recently, it has been shown that there are at least four subclasses of human CSF proteins which can be defined according to the types of cells found in the resultant colonies. One subclass, CSF-1 results in colonies containing macrophages predominantly. Other subclasses produce colonies which contain both neutrophilic granulocytes and macrophages; which contain exclusively neutrophilic granulocytes; and which contain neutrophilic and eosinophilic granulocytes and macrophages.

The invention herein is concerned with the purification of proteins which are members of the first of these subclasses, CSF-1. This subclass has been further characterized and delineated by specific radioimmunoassays and radioreceptor assays -- e.g., antibodies raised against purified CSF-1 are able to suppress specifically CSF-1 activity, without affecting the biological activities of the other subclasses, and macrophage cell line J774 contains receptors which bind CSF-1 specifically. A description of these assays was published by Das, S.K., et al, Blood (1981) 58:630.

Purification methods for various CSF proteins have been published.

Stanley, E.R., et at, J Biol Chem (1977) 252:4305-4312 disclosed a purification procedure for CSF-1 from human urine and Das, S.K., et at, Blood (1981) 58:630; J Biol Chem (1982) 257:13679 obtained a human urinary CSF-1 at a specific activity of 5 x 10$^7$ units/mg which produced only macrophage colonies, and outlined the relationship of glycosylation of the CSF-1 proteins prepared from cultured mouse L-cells and from human urine to their activities. Wang, F.F., et at, J Cell Biochem (1983) 21:263, isolated human urinary CSF-1 to specific activity of 10$^8$ U/mg. Waheed, A., et at, disclosed purification of human urinary CSF-1 to a specific activity of 0.7-2.3 x 10$^7$ U/mg on a rabbit antibody column (Exp Hemat (1984) 12:434).

Wu, M., et al, J Biol Chem (1979) 254:6226 reported the preparation of a CSF protein from cultured human pancreatic carcinoma (MIAPaCa) cells which resulted in the growth of murine granulocytic and macrophagic colonies. The resulting protein had a specific activity of approximately 7 x 10$^7$ units/mg. Partially purified preparations of various CSFs have also been reported from human and mouse lung-cell conditioned media (Fojo, S.S., et at, Biochemistry (1978) 17:3109; Burgess, A.W., et at, J Biol Chem (1977) 252:1998); from human T-lymphoblast cells (Lusis, A.J., et at, Blood (1981) 57:13; U.S. Patent, 4,438,032); from human placental conditioned medium to apparent homogeneity and specific activity of 7 x 10$^7$ U/mg (Wu, M., et at, Biochemistry (1980) 19:3846). a different subclass, murine and human GM-CSF, has been purified and the cDNAs cloned. This protein was shown to be distinct from other CSFs, e.g., CSF-1, by Gough, et al, Nature (1984) 309:763-767. Murine IL-3 has been cloned by Fung, M. C., et al, Nature (1984) 307:233. See also Yokota, T., et al, PNAS (1984) 81:1070-1074; Wong, G.G., et al, Science (1985) 228:810-815; Lee, F., et al, PNAS (1985) 82:4360-4364; and Cantrell, M.A., et al, PNAS (1985) 82:6250-6254.

In one aspect, the present invention relates to a purified human CSF-1 preparation having the N-terminal amino acid sequence comprising Glu-Glu-Val-Ser-Glu-Tyr-Cys-Ser-His-Met-Ile-Gly-Ser-Gly-His-Leu-Gln-Ser-Leu-Gln-Arg-Leu-Ile-Asp-Ser-Gln-Met-Glu-Thu-Ser-Cys-Gln-Ile-Thr-Phe-Glu-Phe-Val-Asp-Gln-Glu-Gln-Leu and dimers of said CSF-1 having the ability to stimulate bone marrow cells in vitro to form primarily macrophage colonies wherein said CSF-1 preparation is obtainable by the process comprising the steps of:

(a) treating a solution containing said CSF-1 with an immunoaffinity column comprising antibodies produced by the hybridoma YYG106 obtainable from HB9014; and
(b) subjecting the resultant CSF-1 containing fractions to reversed-phase HPLC wherein said reversed-phase HPLC employs a 0.1% trifluoroacetic acid/30% acetonitrile to 0.1% trifluoroacetic acid/60% acetonitrile buffer system.

Refinements in purification techniques and careful sequencing have made possible identification of N-terminal sequences of human CSF-1. This permits construction of probes useful in analyzing disease states, and in assessing changes in CSF-1 protein associated with these disease states. The probes are also useful as tools in obtaining DNA encoding CSF-1 for use in devising recombinant production of CSF-1 protein.

EP 0 211 899 B1

Thus, in another aspect, the invention relates to probes designed on the basis of the determined sequences.

In another aspect, the invention relates to an improved method to purify CSF-1 from humans. This method includes use of immunoaffinity chromatography to effect an efficient specific purification step followed by the use of reverse phase HPLC to assure freedom from contaminants. Monoclonal antibodies are used in the immunoaffinity chromatography step. The invention thus provides a process for preparing a purified human CSF-1 which comprises:-

(a) treating a solution containing said CSF-1 with an immunoaffinity column comprising antibodies produced by the hybridoma YYG106 obtainable from HB9014; and

(b) subjecting the resultant CSF-1 containing fractions to reversed-phase HPLC wherein said reversed-phase HPLC employs a 0.1% trifluoroacetic acid/30% acetonitrile to 0.1% trifluoroacetic acid/60% acetonitrile buffer system.

In another aspect the invention provides the use of a CSF-1 preparation as defined above in the manufacture of a medicament.

The invention further provides a pharmaceutical formulation comprising a CSF-1 preparation as defined above formulated for pharmaceutical use, optionally with at least one additional component.

In the accompanying drawings:-

Figure 1 shows the partial amino acid sequence of human urinary and MIAPaCa CSF-1 as determined from purified native proteins.

Figure 2 shows the seqence of oligomer probes designed from the amino acid sequence of human CSF-1.

A. Definitions

"Colony stimulating factor-1 (CSF-1)" refers to a protein which exhibits the spectrum of activity understood in the art for CSF-1 -- i.e., when applied to the standard in vitro colony stimulating assay of Metcalf, D., J Cell Physiol (1970) 76:89, it results in the formation of primarily macrophage colonies. This factor is also active in the bone marrow proliferation assays of Moore, R.N., et al, J Immunol (1983) 131:2374, and of Prystowsky, M.B., et al, Am J Pathol (1984) 114:149. This protein may be isolated from any vertebrate species, but there appears to be some species specificity: Human CSF-1 is operative both on human and on murine bone marrow cells; murine CSF-1 does not show activity with human cells. Therefore, human CSF-1 should be positive in the specific murine radioreceptor assay of Das, S.K., et al, Blood (1981) 58:630, and the biological activity of the human protein is inhibited by neutralizing antiserum to human urinary CSF-1 (Das, S.K., et al, supra).

Certain other properties of CSF-1 have been recognized more recently, including the ability of this protein to stimulate the secretion of series E prostaglandins, interleukin-1, and interferon from mature macrophages (Moore, R., et al, Science (1984) 223:178) and other effects on monocytes as described below. The mechanism for these latter activities is not at present understood, and for purposes of definition herein, the criterion for fulfillment of the definition resides in the ability to stimulate the formation of monocyte/macrophage colonies using bone marrow cells from the appropriate species as starting materials. (It is known that the proliferative effect of CSF-1 is restricted to cells of mononuclear phagocytic lineage (Stanley, E.R., The Lymphokines (1981), Stewart, W.E., II, et al, ed, Humana Press, Clifton, NJ), pp. 102-132) and that receptors for CSF-1 are restricted to these cell lines (Byrne, P.V., et al, Cell Biol (1981) 91:848)).

As is the case for all proteins, the precise chemical structure depends on a number of factors. As ionizable amino and carboxyl groups are present in the molecule, a particular protein may be obtained as an acidic or basic salt, or in neutral form. All such preparations which retain their activity when placed in suitable environmental conditions are included in the definition. Further, the primary amino acid sequence may be augmented by derivatization using sugar moieties (glycosylation) or by other supplementary molecules such as lipids, phosphate, acetyl groups and the like, more commonly by conjugation with saccharides. The primary amino acid structure may also aggregate to form complexes, most frequently dimers. Indeed, native human urinary CSF-1 is isolated as a highly glycosylated dimer of 45 kd. Certain aspects of such augmentation are accomplished through post-translational processing systems of the producing host; other such modification may be introduced in vitro. In any event, the subject protein is within the definition of CSF-1 regardless of its state of aggregation or derivatization so long as the activity of the protein, as defined above, is present. It is expected, of course, that such modifications may quantitatively or qualitatively affect the activity, either by enhancing or diminishing the activity of the protein in various assays.

4

Further, individual amino acid residues in the chain may be modified by oxidation, reduction or other derivatization at the protein level, or the protein may be cleaved to obtain active fragments. Such alterations which do not destroy activity are included in the definition.

B. Utility

The CSF-1 proteins of the invention are capable both of stimulating monocyte-precursor/macrophage cell production from progenitor marrow cells, thus enhancing the effectiveness of the immune system, and of stimulating such functions of these differentiated cells as the secretion of lymphokines in the mature macrophages.

In one application, these proteins are useful as adjuncts to chemotherapy. It is well understood that chemotherapeutic treatment results in suppression of the immune system. Often, although successful in destroying the tumor cells against which they are directed, chemotherapeutic treatments result in the death of the subject due to this side effect of the chemotoxic agents on the cells of the immune system. Administration of CSF-1 to such patients, because of the ability of CSF-1 to mediate and enhance the growth and differentiation of bone marrow-derived precursors into macrophages, results in a restimulation of the immune system to prevent this side effect, and thus to prevent the propensity of the patient to succumb to secondary infection. Other patients who would be helped by such treatment include those being treated for leukemia through bone marrow transplants; they are often in an immunosuppressed state to prevent rejection. For these patients also, the immunosuppression could be reversed by administration of CSF-1.

In general, any subject suffering from immunosuppression whether due to chemotherapy, bone marrow transplantation, or other, accidental forms of immunosuppression such as disease (e.g., acquired immune deficiency syndrome) would benefit from the availability of CSF-1 for pharmacological use. In addition, subjects could be supplied enhanced amounts of previously differentiated macrophages to supplement those of the indigenous system, which macrophages are produced by in vitro culture of bone marrow or other suitable preparations treated with CSF-1. These preparations include those of the patient's own blood monocytes, which can be so cultured and returned for local or systemic therapy.

The ability of CSF-1 to stimulate production of lymphokines by macrophages also makes CSF-1 directly useful in treatment of neoplasms and infections.

CSF-1 stimulates the production of interferons by murine-derived macrophage (Fleit, H.B., et al, J Cell Physiol (1981) 108:347, and human, partially purified, CSF-1 from MIAPaCa cells stimulates the poly IC-induced production of interferon and TNF from human monocytes as illustrated below. In addition, CSF-1 stimulates the production of myeloid CSF by human blood monocytes.

In addition to overcoming immunosuppression per se, CSF-1 can be used to destroy invading organisms or malignant cells indirectly by stimulation of macrophage secretions and activity.

The CSF-1 of the invention may be formulated in conventional ways standard in the art for the administration of protein substances. Administration by injection is preferred; formulations include solutions or suspensions, emulsions, or solid composition for reconstitution into injectables. Suitable excipients include, for example, Ringer's solution, Hank's solution, water, saline, glycerol, dextrose solutions, and the like. In addition, the CSF-1 of the invention may be preincubated with preparations of cells in order to stimulate appropriate responses, and either the entire preparation or the supernatant therefrom introduced into the subject. As shown hereinbelow, the materials produced in response to CSF-1 stimulation by various types of blood cells are effective against desired targets, and the properties of these blood cells themselves to attack invading viruses or neoplasms may be enhanced. The subject's own cells may be withdrawn and used in this way, or, for example, monocytes or lymphocytes from another compatible individual employed in the incubation.

Although the existence of a pattern of activity designated CSF-1 has been known for some time, sufficient amounts of the protein responsible have never been obtained in sufficiently pure form to permit sequence determination, thus the construction of DNA probes to study disease states based on their associated nucleic acid patterns for lymphokine encoding materials has not been possible. The present invention provides sufficient sequence information so that probes can be constructed. Through a variety of additional purification procedures, sufficient pure CSF-1 has been obtained from human urine and from MIAPaCa cells to provide some amino acid sequence, thus permitting the construction of DNA oligomeric probes. The probes are useful in obtaining the coding sequence for the entire protein, as well as for assessing disease states. The purified protein, of course, is also useful therapeutically as described above, and for the production of antibodies for diagnostic and therapeutic use.

C. Purification

5

The CSF-1 proteins of the invention were purified in sufficient homogeneity and quantity to obtain N-terminal sequence in several ways.

As illustrated below, human urinary CSF-1 was partially purified by standard methods as described by Das, S. K., et al, Blood (1981) 58:630. followed by an affinity purification step using a rat monoclonal antibody to murine CSF-1, designated YYG106, attached to a Sepharose B column (Stanley, E.R., Methods Enzymol (1985) 116:564). The final step in purification was reverse phase HPLC in a 0.1% TFA/30% acetonitrile - 0.1% TFA/60% acetonitrile buffer system.

For MIAPaCa CSF-1, which was produced serum-free by induction with phorbol myristic acetate, the cell superatant was subjected to calcium phosphate gel chromatography (according to Das (supra)), followed by affinity chromatography using lentil lectin (in place of the ConA affinity step of Das), and then to the immunoaffinity step employing the YYG106 monoclonal antibody conjugated to Sepharose B and to the reverse phase HPLC.

In general, purification procedures for CSF-1 protein are particularly effective which utilize an immunoaffinity chromatography step, preferably, an immunoaffinity step employing a monoclonal antibody preparation, followed by reverse phase HPLC. The proteins may also be further analyzed using SDS-PAGE.

Immunoaffinity chromatography involves the use of standard methods, whereby the antibody preparation is supported on a suitable polymer support such as Sepharose®, dextran, or polyacrylamide, employing procedures appropriate to the nature of the support. Polyclonal antibody preparations for use in this step are prepared by immunizing a subject, preferably a mammalian subject, such as a rabbit, mouse, or rat with the purified protein, such as that derived from human urine The antiserum may be used directly as a polyclonal composition, or the spleen cells or peripheral blood lymphocytes of the immunized subject may be immortalized using, for example, the fusion procedure of Kohler and Milstein. The successfully fused cells are then screened for production of antibodies against CSF-1 to obtain a monoclonal antibody producing line. Monoclonal preparations are, of course, preferred, as a consistent composition is more easily obtained. A particularly preferred monoclonal antibody is that produced by the YYG106 cell line, which is a fusion between a rat myeloma line and spleen cells from a rat immunized with murine L-cell CSF-1.

For reverse phase HPLC, standard techniques are also employed. Any hydrophobic column, such as an alkyl-, aryl-, alkylaryl, or arylalkyl derivatized support, for example phenyl sepharose or phenyl TSK may be used. The elution gradient depends on the choice of support.

Amino acid composition determination and sequencing were done by standard procedures, however the procedures were adapted to the specific problems presented by the proteins available, as further described below.

## D. Probe Construction

Using the sequence information obtained from the purified proteins above, oligomeric DNA sequences were constructed using standard, commercially available techniques. Codon redundancy is accounted for by using mixtures of probes, or by using limited numbers of particular oligomers which include codons favored in mammalian expression.

## E. Examples

The following examples are intended to illustrate but not to limit the invention.

### E.1. Purification of Native Human CSF-1

Human urinary CSF-1 was partially purified by standard methods as described by Das, S. K., et al, Blood (1981) 58:630, followed by an affinity purification step using a rat monoclonal antibody to murine CSF-1, designated YYG106, attached to a Sepharose B column (Stanley, E.R., Methods Enzymol (1985) 116:564). The final step in purification was reverse phase HPLC in a 0.1% TFA/30% acetonitrile - 0.1% TFA/60% acetonitrile buffer system.

For MIAPaCa CSF-1, which was produced serum-free by induction with phorbol myristic acetate, the cell superatant was subjected to calcium phosphate gel chromatography (according to Das (supra)). followed by affinity chromatography using lentil lectin (in place of the ConA affinity step of Das), and then to the immunoaffinity step employing the YYG106 monoclonal antibody conjugated to Sepharose B and to the reverse phase HPLC, both as above described.

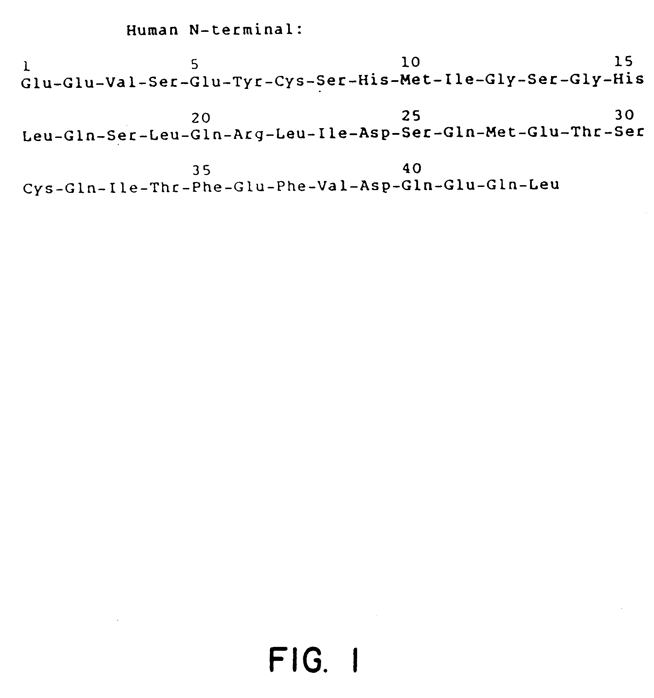

The urinary and MIAPaCa proteins, having been purified to homogeneity, were subjected to amino acid sequencing using Edman degradation on an automated sequencer. Sufficient N-terminal sequence of human CSF was determined (Figure 1) to permit construction of the probes shown in Figure 3.

In more detail, for both MIAPaCa and urinary CSF-1, all buffers used contain 3 mM $NaN_3$ and 0.01 g/l PEG-6000. The initial step in each case is DEAE cellulose chromatography. About 100 1 of pooled urine or an amount of MIAPaCa medium containing a comparable amount of CSF-1 activity are adjusted to pH 7.4 and dialyzed to remove salts. The dialyzate is then applied to a DEAE cellulose column (200 g dry weight, Eastman) preequilibrated in 30 mM Tris-HCl buffer. pH 7.4.

The column is washed with 40 mM NaCl in the same buffer, and eluted with the same buffer containing 250 mM NaCl. The fractions containing CSF-1, as analyzed by the bone marrow proliferation assay, are dialyzed or ultrafiltered to remove ions. The deionized eluate is then treated with calcium phosphate gel (58 ml/g protein) and the gel is washed twice by decantation with 10 l of 5 mM sodium phosphate buffer, pH 6.5. The slurry is resuspended in 2.5 l and adjusted to 25 mM sodium phosphate buffer, pH 6.5 to elute the CSF-1, which is separated from the gel by centrifugation at 12,000 x g for 10 min and concentrated to 50 ml for further DEAE cellulose chromatography.

The eluted CSF-1 in 100 mM Tris-HCl buffer, pH 7.4 is then applied to a DEAE cellulose column preequilibrated with the same buffer and eluted with a linear gradient of NaCl (0-150 mM) in the same buffer. The CSF-1 elutes at approximately 75-130 mM NaCl, and these fractions are dialyzed and concentrated to 15 ml for affinity chromatography on ConA Sepharose.

The concentrate is dissolved in 100 mM acetate, l M NaCl, 10 mM $MgCl_2$, 10 mM $CaCl_2$, 10 mM $MnCl_2$, pH 6.0 (ConA buffer) and applied to a ConA Sepharose column (Pharmacia). The column is washed with ConA buffer at 4°C and then eluted with 100 mM $\alpha$-methyl-D-glucoside in the same buffer and the fractions containing CSF-1, as determined by the bone marrow proliferation assay, are pooled and concentrated to 3 ml for gel filtration.

The CSF-1 is taken up in 30 mM Tris-HCl, pH 7.4 and applied to a Biogel® P-100 column equilibrated in the same buffer. The active fractions elute during the void volume and are pooled and dialyzed against 50 mM phosphate, pH 6.5.

The pooled eluate from the gel filtration step is then subjected to immunosorbent chromatography using $10^5$ U CSF-1 for each ml of a PABAE-Seph-4B column derivatized to anti-CSF-1 monoclonal antibody, prepared as described in the following paragraph.

(The column was prepared using the monoclonal antibody YYG106 which is produced from a hybridoma maintained in suspension culture of 10% FCS-$\alpha$ medium. The hybridoma was obtained by fusion between spleen cells from a rat immunized with partially purified murine L-cell CSF-1 and a rat myeloma line. Serum-free medium for the production of the desired monoclonal antibody is prepared by culturing washed cells ($10^5$/ml) in HB101 medium (Hana Biologics, Berkeley, CA) and harvesting the medium by centrifugation at 400 x g for 15 min at 4°C from cultures in which cell viability has dropped to 25%. The recovered medium is then brought to 50% saturation with ammonium sulfate and the precipitate collected by centrifugation at 1200 g for 15 min at 4°C, dialyzed against 20 mM sodium phosphate buffer, pH 7.1 and applied to a DEAE Affigel® Blue (BioRad Labs, Rockville Center, NY) column equilibrated in the same buffer at 4°C. The column is then washed with 20 mM sodium phosphate buffer, pH 7.1 and the desired monoclonal antibody eluted with a 0-0.15 M NaCl gradient in this buffer. The antibody activity of the fractions is determined and the active fractions pooled and concentrated by ultrafiltration.

A packed bed volume of PABAE-Seph (Sepharose 4B derivatized with p-aminobenzamidoethyl-) is washed in 0.5 M HCl (cold) and treated with 0.2 M $NaNO_2$ incubated on ice for 7 min. The gel is washed 3 times with at least 2 volumes of ice cold distilled water and equal volumes of washed gel and concentrated monoclonal antibody (3 mg/ml in 0.2 M sodium borate buffer, pH 8.0) are mixed and rocked for 16 hr at 4°C. The resulting derivatized PABAE-Seph-4B is then washed sequentially with 5 volumes of 1% triethanolamine in 50 mM Tris-HCl, pH 8.5, 5 volumes of 6 M urea in 0.1 M Tris-HCl, pH 7.4 and 5 volumes of 0.4 M sodium bicarbonate prior to equilibration in 0.05 M sodium phosphate buffer, pH 6.5.)

The pooled gel filtration eluate as described above is treated with the PABAE-Seph-4B-antibody derivatized column at $10^5$ U/ml at 4°C, and recycled through the column. The column is washed with 50 mM sodium phosphate buffer, pH 6.5, then with 100 mM glycine-HCl, pH 2.0 prior to elution using 4 M KSCN, and then 0.1 M glycine-HCl, pH 2.0, which latter eluate is collected in a vessel containing 0.6 column volumes of 1 M ammonium bicarbonate. The eluates are separately dialyzed against 0.01 g/l PEG-6000.

The resulting human urinary CSF-1 has a specific activity of approximately 8 x $10^7$ U/mg.

The human urinary or MIAPaCa CSF-1 is then subjected to reverse phase HPLC using 0.1% TFA/acetonitrile gradient as described above.

For some MIAPaCa preparations, derived from serum-free medium, the purification is accomplished using the calcium phosphate gel filtration step as described above, followed by affinity chromatography using lentil lectin in place of ConA, but otherwise as described above, followed by the immunoabsorbant chromatography and HPLC steps described.

SDS-gel electrophoresis of the HPLC eluates confirms homogeneity for both human urinary and MIAPaCa preparations.

Construction of Probes

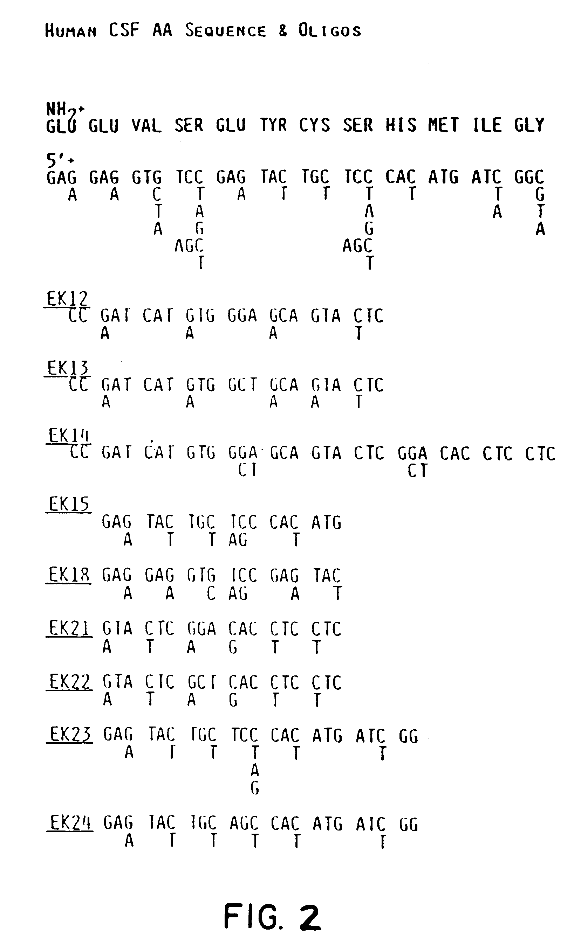

Sufficient N-terminal sequence of human CSF was determined to permit construction of probes as shown in Figure 2. The N-terminal sequences of the purified MIAPaCa and urinary CSF-1 are identical. The resulting synthetic oligonucleotides are useful for diagnosis and determining the etiology of various disease states in humans.

E.2. Biological Activity

Additional data relevant to the activity of CSF-1 was provided using partially purified MIAPaCa CSF-1. CSF-1 was shown to enhance the production of interferon and tumor necrosis factor (TNF) by induced human monocytes by up to 10-fold. CSF-1 also was demonstrated to stimulate macrophage antitumor toxicity.

Stimulation of TNF Production by Human Monocytes

MIAPaCa CSF-1 was purified from the supernatant by calcium phosphate gel filtration and lentil lectin chromatography. For assay of lymphokine production, peripheral blood-adherent cells were incubated in duplicate flasks containing $10^7$ cells each. One flask was treated with 1000 U/ml CSF-1 purified as above. After 3 days, the cells were harvested, and washed, and resuspended at a cell concentration of $5 \times 10^5$/ml and plated in 24-well plates at 0.5 ml/well. The wells were treated with 10 $\mu$g/ml LPS and 20 ng/ml PMA for 48 hr and the supernatants were harvested for TNF assay. Cells treated with CSF showed TNF secretions approximately nine-fold higher than the untreated cells (1500 U/ml, compared to 162 U/ml).

Stimulation of Interferon Production by Human Monocytes

In an analogous experiment to determine the effect of CSF-1 on interferon production, peripheral blood-adherent cells were incubated for 3 days in the presence and absence of 1000 U/ml CSF-1, as described above, harvested, resuspended at $5 \times 10^5$/ml, and plated in a 25-well plate, as described above. The cells were induced for interferon production by addition of varying amounts of poly IC. The supernatants were assayed for interferon production by their cytopathic effect on VSV-infected GM 2504 cells. The CSF-1-stimulated cells showed production of 100 U/ml when induced with 50 ug/ml poly IC, as described by McCormick, F., et al, Mol Cell Biol (1984) 4:166, whereas comparably induced untreated cells produced less than 3 U/ml.

Stimulation of Myeloid CSF Production by Human Monocytes

Monocytes were incubated ± CSF-1 for 3 days and then induced for production of myeloid CSF as in Table 1. The three representative experiments shown, used blood from different donors.

Table 1

| Myeloid CSF (U/ml) | | | | | | |
|---|---|---|---|---|---|---|
| Induction | Exp. 1 | | Exp. 2 | | Exp. 3 | |
| | -CSF | +CSF | -CSF | +CSF | -CSF | +CSF |
| medium | 0 | 0 | 0 | 0 | 0 | 0 |
| 0.1 $\mu$g/ml LPS | - | - | 0 | 0 | 0 | 80±17 |
| 1 $\mu$g/ml LPS | 0 | 700±72 | 40±20 | 200±20 | 103±12 | 377±57 |
| 0.1 $\mu$g/ml LPS + 2 ng/ml PMA | - | - | 617±50 | 993±101 | 1120±82 | 1280±60 |
| 1 $\mu$g/ml LPS + 2 ng/ml PMA | 283±42 | 983±252 | 360±92 | 1400±180 | 537±47 | 1080±122 |
| 2 ng/ml PMA | - | 370±17 | 297±6 | 183±15 | 380±52 | 716±76 |

Therefore, CSF-1 stimulates CSF-GM production.

The possibility of contaminating bacterial lipopolysaccharide (LPS) as the cause of stimulation of monocytes and macrophages was excluded: The LPS content of the applied CSF-1 was low (<0.3 ng/3000 U CSF-1, by Limulus amoebocyte lysate assay); activity was removed by application to an anti-CSF-1 column; polymyxin B was used to neutralize LPS; macrophages from C3H/HeJ mice respond to CSF-1 but do not respond to LPS.

Stimulation of Murine Antiviral Activity

Adherent murine thioglycolate-elicited macrophages were incubated with CSF-1 for 3 days and infected with VSV overnight. Polymyxin B was added to test samples to block the LPS induction of interferon. The following table shows crystal violet staining of cells remaining adherent.

Table 2

| Treatment | Crystal Violet | |
|---|---|---|
| | -Polymyxin B (mean)(S.D.) | +Polymyxin B |
| Medium/No VSV | .158±.019 | |
| Medium + VSV | .0583±.02 | .049±.009 |
| CSF-1625 U/ml + VSV | .139±.018 | .177±.04 |
| 1250 + VSV | .167±.06 | .205±.07 |
| 2500 + VSV | .160±.06 | .219±.04 |
| 5000 + VSV | .150±.03 | .202±.06 |

CSF-1 treated cells, therefore, showed protection of the macrophage against VSV.

E.3. Formulation of CSF-1

The recombinantly produced human CSF-1 may be formulated for administration using standard pharmaceutical procedures. Ordinarily CSF-1 will be prepared in injectable form, and may be used either as the sole active ingredient, or in combination with other proteins or other compounds having complementary or similar activity. Such other compounds may include alternate antitumor agents such as adriamycin, or lymphokines, such as IL-1, -2, and -3, alpha-, beta-, and gamma-interferons and tumor necrosis factor. The effect of the CSF-1 active ingredient may be augmented or improved by the presence of such additional components. As described above, the CSF-1 may interact in beneficial ways with appropriate blood cells, and the compositions of the invention therefore include incubation mixtures of such cells with CSF-1, optionally in the presence of additional lymphokines. Either the supernatant fractions of such incubation mixtures, or the entire mixture containing the cells as well may be used.

**Claims**

**1.** A purified human CSF-1 preparation having the N-terminal amino acid sequence comprising Glu-Glu-Val-Ser-Glu-Tyr-Cys-Ser-His-Met-Ile-Gly-Ser-Gly-His-Leu-Gln-Ser-Leu-Gln-Arg-Leu-Ile-Asp-Ser-Gln-Met-Glu-Thr-Ser-Cys-Gln-Ile-Thr-Phe-Glu-Phe-Val-Asp-Gln-Glu-Gln-Leu and dimers of said CSF-1 having the ability to stimulate bone marrow cells in vitro to form primarily macrophage colonies wherein said CSF-1 preparation is obtainable by the process comprising the steps of:
a) treating a solution containing said CSF-1 with an immunoaffinity column comprising antibodies produced by the hybridoma YYG106 obtainable from HB9014; and
b) subjecting the resultant CSF-1 containing fractions to reversed-phase HPLC wherein said reversed-phase HPLC employs a 0.1% trifluoroacetic acid/30% acetonitrile to 0.1% trifluoroacetic acid/60% acetonitrile buffer system.

**2.** A human CSF-1 preparation as claimed in Claim 1, wherein said CSF-1 preparation is obtainable by a process further comprising the step of affinity chromatography on Con A-Sepharose preceding said immunoaffinity column.

3. A process for preparing a purified human CSF-1 which comprises:-
   a) treating a solution containing said CSF-1 with an immunoaffinity column comprising antibodies produced by the hybridoma YYG106 obtainable from HB9014; and
   b) subjecting the resultant CSF-1 containing fractions to reversed-phase HPLC wherein said reversed-phase HPLC employs a 0.1% trifluoroacetic acid/30% acetonitrile to 0.1% trifluoroacetic acid/60% acetonitrile buffer system.

4. A process as claimed in Claim 3 further comprising the step of affinity chromatography on Con A-Sepharose preceding said immunoaffinity column.

5. A DNA fragment comprising the sequence selected from the group consisting of:

```
GAACATTGT TCA CA
   G   C   C    T
                G
                C;


GAACATTGT AGT CA
   G   C   C    C;


CATATGATTGGAAAT
     C      C  T  C
            A  G
               C;


TG AGA  ACAATGTTC
   T    G   G   C
   G
   C;


TG ACT  ACAATGTTC
   G    G   G   C;        and


ATTACCAATCATATG
G   T   G       G
G
C.
```

6. A DNA fragment comprising the sequence selected from the group consisting of the DNA sequences set forth in Figure 2.

7. A method to enhance the production of a GM-CSF or TNF or interferon from monocytes in vitro which comprises treating said monocytes with an effective amount of a CSF-1 preparation as defined in Claim 1 or Claim 2.

8. A method to enhance killing of target cells by macrophage in vitro which comprises treating said macrophages with an effective amount of a CSF-1 preparation as defined in Claim 1 or Claim 2.

9. A method to induce resistance to viral infections in macrophages in vitro which comprises treating said macrophages with an effective amount of a CSF-1 preparation as defined in Claim 1 or Claim 2.

10. The use of a CSF-1 preparation as defined in Claim 1 or Claim 2 in the manufacture of a medicament.

11. The use of Claim 10, wherein the medicament is for enhancement of the immune system of a subject.

12. A pharmaceutical formulation comprising a CSF-1 preparation as claimed in Claim 1 or Claim 2 formulated for pharmaceutical use, optionally with at least one additonal component.

13. A composition as claimed in Claim 12, wherein the additional component is a pharmaceutical excipient, a cell culture supernatant, and/or a cell culture.

**Patentansprüche**

1. Gereinigte menschliche CSF-1-Präparation, die die N-terminale Aminosäuresequenz Glu-Glu-Val-Ser-Glu-Tyr-Cys-Ser-His-Met-Ile-Gly-Ser-Gly-His-Leu-Gln-Ser-Leu-Gln-Arg-Leu-Ile-Asp-Ser-Gln-Met-Glu-Thr-Ser-Cys-Gln-Ile-Thr-Phe-Glu-Phe-Val-Asp-Gln-Glu-Gln-Leu aufweist, und Dimere des CSF-1, die die Fähigkeit aufweisen, Knochenmarkszellen in vitro sur Bildung von haupsächlich Makrophagenkolonien Zu stimulieren, wobei die CSF-1-Präparation durch das Verfahren erhältlich ist, das die folgenden Schritte umfaßt:
   a) Behandeln einer den CSF-1 enthaltenden Lösung mit einer Immunaffinitäts-Säule, die Antikörper enthält, die von dem aus HB9014 erhältlichen Hybridom YYG106 produziert werden; und
   b) Unterwerfen der CSF-1 enthaltenden Fraktionen einer Revers-Phasen-HPLC, wobei in der Revers-Phasen-HPLC ein Puffer-System von 0,1 % Trifluoressisäure/30 % Acetonitril bis 0,1 % Trifluoressigsäure/60 % Acetonitril verwendet wird.

2. Menschliche CSF-1-Präparation nach Anspruch 1, wobei die CSF-1-Präparation durch ein Verfahren erhältlich ist, das als zusätzlichen Schritt vor der Immunaffinitäts-Säule eine Affinitäts-Chromatographie an Con A-Sepharose umfaßt.

3. Verfahren zur Herstellung eines gereinigten menschlichen CSF-1, das umfaßt:
   a) Behandeln einer den CSF-1 enthaltenden Lösung mit einer Immunaffinitäts-Säule, die Antikörper enthält, die von dem aus HB9014 erhältlichen Hybridom YYG106 produziert werden; und
   b) Unterwerfen der CSF-1 enthaltenden Fraktionen einer Revers-Phasen-HPLC, wobei in der Revers-Phasen-HPLC ein Puffer-System von 0,1 % Trifluoressisäure/30 % Acetonitril bis 0,1 % Trifluoressigsäure/60 % Acetonitril verwendet wird

4. Verfahren nach Anspruch 3, das als zusätzlichen Schritt vor der Immunaffinitäts-Säule eine Affinitäts-Chromatographie an Con A-Sepharose umfaßt.

5. DNA-Fragment, das eine Sequenz enthält, die aus der Gruppe folgender Sequenzen ausgewählt ist:

```
GAACATTGT  TCA  CA
  G    C   C    T
               G
               C,

GAACATTGT  AGT  CA
  G    C   C    C,

CATATGATTGGAAAT
    C       C  T  C
            A  G
               C,

TG  AGA  ACAATGTTC
  T    G    G   C
  G
  C,

TG  ACT  ACAATGTTC
   G    G    G   C        und

ATTACCAATCATATG
  G  T  G     G
  G
  C
```

**6.** DNA-Fragment, das eine Sequenz enthält, die aus den in Figur 2 gezeigten DNA-Sequenzen ausgewählt ist.

**7.** Verfahren zur Steigerung der Produktion eines GM-CSF oder TNF oder Interferons aus Monocyten in vitro, das eine Behandlung der Monocyten mit einer wirksamen Menge einer CSF-1-Präparation, wie sie in einem der Ansprüche 1 oder 2 definiert ist, umfaßt.

**8.** Verfahren zur Steigerung der Abtötung von Zielzellen durch Makrophagen in vitro, das eine Behandlung der Makrophagen mit einer wirksamen Menge einer CSF-1-Präparation, wie sie in einem der Ansprüche 1 oder 2 definiert ist, umfaßt.

**9.** Verfahren zur Induktion einer Resistenz in Makrophagen in vitro gegen virale Infektionen, das eine Behandlung der Makrophagen mit einer wirksamen Menge einer CSF-1-Präparation, wie sie in einem der Ansprüche 1 oder 2 definiert ist, umfaßt.

**10.** Verwendung einer CSF-1-Präparation, wie sie in einem der Ansprüche 1 oder 2 definiert ist, zur Herstellung eines Arzneimittels.

**11.** Verwendung nach Anspruch 10, wobei das Medikament zur Verbesserung des Immunsystems eines Organismus dient.

**12.** Arzneimittel-Formulierung, enthaltend eine für die pharmazeutische Verwendung formulierte CSF-1-Präparation, wie sie in einem der Ansprüche 1 oder 2 beansprucht wird, gegebenenfalls mit mindestens einem zusätzlichen Bestandteil.

**13.** Formulierung nach Anspruch 12, wobei der zusätzliche Bestandteil ein Arzneimittelträger, ein Zellkulturüberstand und/oder eine Zellkultur ist.

**Revendications**

1. Préparation de CSF-1 humain purifié ayant la séquence d'acides aminés N-terminale comprenant Glu-Glu-Val-Ser-Glu-Tyr-Cys-Ser-His-Met-Ile-Gly-Ser-Gly-His-Leu-Gln-Ser-Leu-Gln-Arg-Leu-Ile-Asp-Ser-Gln-Met-Glu-Thr-Ser-Cys-Gln-Ile-Thr-Phe-Glu-Phe-Val-Asp-Gln-Glu-Gln-Leu et les dimères du dit CSF-1 ayant la capacité de stimuler les cellules de moelle osseuse in vitro pour former principalement des colonies de macrophages, la dite préparation de CSF-1 pouvant être obtenue par le procédé comprenant les étapes de :

    (a) Traitement d'une solution contenant les dits CSF-1 sur une colonne d'immunoaffinité contenant des anticorps produits par l'hybridome YYG106 pouvant être obtenu à partir de HB9014; et

    (b) Soumission des fractions obtenues contenant du CSF-1 à l'HPLC en phase inversée, la dite HPLC en phase inversée étant réalisée à l'aide d'un système de tampon acide trifluoroacétique 0,1%/acétonitrile 30% à 0,1% d'acide trifluoroacétique/acétonitrile 60%.

2. Préparation de CSF-1 humain comme revendiqué dans la revendication 1, dans laquelle la dite préparation de CSF-1 peut être obtenue par un procédé comprenant en outre une étape de chromatographie d'affinité sur ConA-Sépharose précédant la dite colonne d'immunoaffinité.

3. Procédé de préparation d'un CSF-1 humain purifié qui comprend :

    (a) le traitement d'une solution contenant les dits CSF-1 sur une colonne d'immunoaffinité contenant des anticorps produits par l'hybridome YYG106 pouvant être obtenu à partir de HB9014; et

    (b) la soumission des fractions obtenues contenant du CSF-1 à l'HPLC en phase inversée, la dite HPLC en phase inversée étant réalisée à l'aide d'un système de tampon acide trifluoroacétique 0,1%/acétonitrile 30% à 0,1% d'acide trifluoroacétique/acétonitrile 60%.

4. Procédé comme revendiqué dans la revendication 3 comprenant en outre l'étape de chromatographie d'affinité sur ConA-Sepharose précédant la dite colonne d'immunoaffinité.

5. Fragment d'ADN comprenant la séquence sélectionnée à partir d'un groupe constitué de :

```
    GAACATTGT TCA CA
      G   C   C   T
                  G
                  C;


    GAACATTGT AGT CA
      G   C   C   C;


    CATATGATTGGAAAT
      C       C  T  C
              A  G
                 C;


    TG AGA ACAATGTTC
       T    G   G   C
       G
       C


    TG ACT ACAATGTTC
       G   G   G  C;     et


    ATTACCAATCATATG
    G  T  G      G
       G
       C.
```

**6.** Fragment d'ADN comprenant la séquence sélectionnée parmi le groupe constitué des séquences d'ADN présentées figure 2.

**7.** Procédé pour améliorer la production d'un GM-CSF ou de TNF ou d'interféron par des monocytes in vitro qui comprend le traitement des dits monocytes par une quantité efficace d'une préparation de CSF-1 comme définie dans la revendication 1 ou la revendication 2.

**8.** Procédé améliorant l'élimination de cellules cibles par des macrophages in vitro, qui comprend le traitement des dits macrophages par une quantité efficace d'une préparation de CSF-1 comme définie dans la revendication 1 ou la revendication 2.

**9.** Procédé induisant la résistance aux infections virales de macrophages in vitro, qui comprend le traitement des dits macrophages par une quantité efficace d'une préparation de CSF-1 comme définie dans la revendication 1 ou la revendication 2.

**10.** Utilisation d'une préparation de CSF-1 comme définie dans la revendication 1 ou la revendication 2 pour la fabrication d'un médicament.

**11.** Utilisation de la revendication 10, dans laquelle le médicament est destiné à l'amélioration du système immunitaire d'un sujet.

**12.** Formulation pharmaceutique comprenant une préparation de CSF-1 comme revendiqué dans la revendication 1 ou la revendication 2, formulée pour une utilisation pharmaceutique, éventuellement avec au moins un composant additionnel.

**13.** Composition comme revendiquée dans la revendication 12, dans laquelle le composant additionnel est un excipient pharmaceutique, un surnageant de culture cellulaire, et/ou une culture cellulaire.

Human N-terminal:

1                5                    10                        15
Glu-Glu-Val-Ser-Glu-Tyr-Cys-Ser-His-Met-Ile-Gly-Ser-Gly-His

                 20                   25                        30
Leu-Gln-Ser-Leu-Gln-Arg-Leu-Ile-Asp-Ser-Gln-Met-Glu-Thr-Ser

                 35                   40
Cys-Gln-Ile-Thr-Phe-Glu-Phe-Val-Asp-Gln-Glu-Gln-Leu

# FIG. I

Human CSF AA Sequence & Oligos

NH₂⁺
GLU GLU VAL SER GLU TYR CYS SER HIS MET ILE GLY

```
5'+
GAG GAG GTG TCC GAG TAC TGC TCC CAC ATG ATC GGC
  A   A   C   T   A   T   T   T   T       T   G
          T   A               A           A   T
          A   G               G               A
             AGC             AGC
              T               T
```

```
EK12
   CC GAT CAT GTG GGA GCA GTA CTC
       A       A       A       T
```

```
EK13
   CC GAT CAT GTG GCT GCA GTA CTC
       A       A       A   A   T
```

```
EK14
   CC GAT CAT GTG GGA GCA GTA CTC GGA CAC CTC CTC
                   CT                  CT
```

```
EK15
     GAG TAC TGC TCC CAC ATG
       A   T   T  AG   T
```

```
EK18 GAG GAG GTG TCC GAG TAC
       A   A   C  AG   A   T
```

```
EK21 GTA CTC GGA CAC CTC CTC
       A   T   A   G   T   T
```

```
EK22 GTA CTC GCT CAC CTC CTC
       A   T   A   G   T   T
```

```
EK23 GAG TAC TGC TCC CAC ATG ATC GG
       A   T   T   T   T       T
                   A
                   G
```

```
EK24 GAG TAC TGC AGC CAC ATG ATC GG
       A   T   T   T   T       T
```

# FIG. 2